# EUROPÄISCHE PATENTSCHRIFT

(11) **EP 0 544 145 B1**
(45) Veröffentlichungstag und Bekanntmachung des Hinweises auf die Patenterteilung: **16.08.1995**
(21) Anmeldenummer: 92119246.4
(22) Anmeldetag: 11.11.1992
(51) Int. Cl.: A61K 6/06

(54) **Dentalkeramischer Werkstoff mit niedriger Verarbeitungstemperatur**
Dental ceramic material processable at low temperature
Matériau céramique dentaire ayant une température de mise en forme peu élevée

(30) Priorität: 27.11.1991 DE 4138875
(43) Veröffentlichungstag der Anmeldung: 02.06.1993
(73) Patentinhaber: Degussa Aktiengesellschaft, 60311 Frankfurt (DE); DUCERA DENTAL-GESELLSCHAFT mbH, 61191 Rosbach (DE)
(72) Erfinder:
(74) Vertreter: Weber, Wolfgang

(56) Entgegenhaltungen:
- EP-A- 0 475 528
- EP-A- 0 478 937
- DE-A- 3 911 460
- FR-A- 2 313 912
- US-A- 4 431 451

## Beschreibung

Die Erfindung betrifft einen dentalkeramischen Werkstoff zur Herstellung und zur Reparatur von metallkeramischem und vollkeramischem Zahnersatz mit einer Verarbeitungstemperatur von unterhalb 700° C und einem Wärmeausdehnungskoeffizienten α von (13-14)·10⁻⁶·K⁻¹ zwischen 20 und 500° C.

Mit Hilfe von dentalkeramischen Werkstoffen ist es möglich, vollkeramischen Zahnersatz herzustellen, an metallkeramischem und vollkeramischem Zahnersatz formverändernde Korrekturen vorzunehmen, beschädigte Teile zu reparieren oder metallischen Zahnersatz mit einer Keramikschicht zu verblenden.

In der Zahnheilkunde werden seit vielen Jahren nach Art einer Emaillierung auf metallischen Gerüsten (Kronen, Brücken) keramische Schichten als Verblendung vorgenommen, um ein natürliches Aussehen des Zahnersatzes zu erreichen. Dabei werden keramische Pulver als wässriger Schlicker auf das metallische Gerüst aufgetragen und bei hohen Temperaturen gebrannt. Dabei ist es wichtig, daß die Brenntemperatur (Verarbeitungstemperatur) der keramischen Masse wenigstens 100° C unterhalb der Solidustemperatur des Werkstoffes des Metallgerüstes liegt und der Wärmeausdehnungskoeffizient der keramischen Masse im Bereich 20 bis 500° C geringfügig kleiner als der des Metallwerkstoffes ist, damit beim Aufbrennen und Abkühlen keine Risse in der Verblendschicht entstehen.

Auf gleiche Weise wie dieser sogenannte metallkeramische Zahnersatz können mit den gleichen keramischen Werkstoffen auf feuerfesten keramischen Modellwerkstoffen auch vollkeramische Zahnersatzstücke, wie Vollkronen, Teilkronen, Einlagefüllungen und Verblendschalen im Sinterverfahren hergestellt werden.

Dentalkeramiken dieser Art benötigen wegen der metallischen Gerüstwerkstoffe beim Verblenden im allgemeinen hohe Wärmeausdehnungskoeffizienten, die zwischen 20 und 500° C bei (13 bis 14)·10⁻⁶·K⁻¹ liegen sollten. Üblicherweise werden diese Keramiken bei 950 ± 30° C verarbeitet, was aber für einige Dentallegierungen zu hoch ist. Es ist sehr schwierig, durch Änderung der Zusammensetzung der Dentalkeramiken die Verarbeitungstemperaturen wesentlich abzusenken, ohne dabei den Wärmeausdehnungskoeffizienten und die Korrosionsfestigkeit negativ zu verändern. Biologisch bedenkliche Bestandteile, wie z.B. Bleioxid, müssen dabei vermieden werden.

Wärmeausdehnungskoeffizienten von (13-14)·10⁻⁶·K⁻¹ zwischen 20 und 500° C erreicht man im allgemeinen nur, wenn die dentalkeramischen Massen 25 bis 30 Gew. % Alkalioxide enthalten. Damit ist aber in saurer Umgebung, wie z.B. in der Mündhöhle unter der Plaque, die Grenze der Korrosionsfestigkeit dieser Keramiken erreicht.

Aus der DE-OS 39 11 460 sind dentalkeramische Werkstoffe zur Herstellung.

Korrektur und Reparatur von metallkeramischem und vollkeramischem Zahnersatz bekannt, die neben SiO₂ als Rest 5-15 % Al₂O₃, 0,5-2,5 % B₂O₃, 0,5-2,5 % Sb₂O₃, 0,1-0,5 % CaO, 0,5-2,5 % BaO, 5-10 % Na₂O, 10-15 % K₂O, 0,1-0,5 % Li₂O und 0,1-0,5 % F₂ enthalten. Diese besitzen zwar einen Wärmeausdehnungskoeffizienten zwischen 20 und 500° C von 13,5 (±1)·10⁻⁶·K⁻¹, ihre Verarbeitungstemperatur von 730 ± 30° C liegt aber für einige Dentallegierungen beim Verblenden noch zu hoch.

In der nicht vorveröffentlichten Patentanmeldung P 40 31 168.6 sind keramische Werkstoffe zum Verblenden von metallischem Zahnersatz beschrieben, die einen Wärmeausdehnungskoeffizienten von (16-17,5)·10⁻⁶·K⁻¹ und eine Verarbeitungstemperatur von 770 ± 70° C aufweisen, Sie bestehen aus 60 bis 68 % SiO₂, 10 bis 15 % Al₂O₃, 0,7 bis 1,5 % B₂O₃, 0 bis 0,5 % Sb₂O₃, 0 bis 0,5 % CeO₂, 0 bis 0,5 % BaO, 0,1 bis 0,5 CaO, 9 bis 12 % K₂O, 9 bis 11 % Na₂O, 0,8 bis 1,4 % Li₂O und 0,2 bis 0,4 % F₂.

Es war daher Aufgabe der vorliegenden Erfindung, einen dentalkeramischen Werkstoff zur Herstellung und zur Reparatur von metallkeramischem und vollkeramischem Zahnersatz zu entwickeln, der einen Wärmeausdehnungskoeffizienten von (13 bis 14)·10⁻⁶·K⁻¹ zwischen 20 und 500° C und eine Verarbeitungstemperatur unterhalb 700° C aufweisen soll. Außerdem sollte er keine biologisch bedenkliche Bestandteile enthalten und im Mund korrosionsbeständig sein.

Diese Aufgabe wird erfindungsgemäß durch einen Werkstoff gelöst, der aus 60 bis 65 Gew. % SiO₂, 8,5 bis 11 Gew. % Al₂O₃, 8 bis 12 Gew. % K₂O, 10,5 bis 12 Gew. % Na₂O, 0,7 bis 2 Gew. % CaO, 0,6 bis 2 Gew. % BaO, 0,5 bis 2,5 Gew. % B₂O₃, 0,1 bis 0,6 Gew. % Sb₂O₃, 0 bis 0,5 Gew. % CeO₂, 1,2 bis 3,8 Gew. % TiO₂, 0,8 bis 1,4 Gew. % Li₂O und 1,2 bis 3,8 Gew. % F₂ besteht.

Vorzugsweise enthalten die dentalkeramischen Massen 60 bis 63 Gew. % SiO₂, 8,5 bis 9,5 Gew. % Al₂O₃, 10 bis 11,5 Gew. % K₂O, 10,5 bis 11,5 Gew. % Na₂O, 0,7 bis 1,5 Gew. % CaO, 0,6 bis 1,2 Gew. % BaO, 0,7 bis 1,5 Gew. % B₂O₃, 0,2 bis 0,4 Gew. % Sb₂O₃, 0,1 bis 0,4 Gew. % CeO₂, 1,5 bis 3 Gew. % TiO₂, 0,8 bis 1,2 Gew. % Li₂O und 1,2 bis 2,4 Gew. % F₂.

Diese erfindungsgemäßen dentalkeramischen Werkstoffe weisen Verarbeitungstemperaturen von 660±30° C auf. Dabei liegen die Glaspunkte bei etwa 450° C und die Erweichungspunkte bei etwa 510° C. Sie besitzen eine sehr hohe Materialhomogenität und eine sehr hohe Transparenz (über 70 % Lichtdurchlässigkeit). Die Biegefestigkeit nach DIN 13925 liegt bei 110 N·mm⁻² und damit weit über deren Mindestanforderung von 50 N·mm⁻².

Der Masseverlust bei der Korrosionsbeständigkeitsprüfung nach DIN 13925 (16 Stunden in 4 %-iger Essigsäure) beträgt etwa 0,028 Gew. % . Dabei wird die Biegefestigkeit überraschenderweise um bis zu 50 % erhöht, während sie bei den bekannten Dentalkeramiken bis zu 30 % abnimmt.

Die folgende Tabelle gibt die Zusammensetzung einiger besonders vorteilhafter Werkstoffe wieder:

| Werkstoff | 1 | 2 | 3 |
|---|---|---|---|
| SiO₂ | 61,3 % | 60,9 | 62,3 |
| Al₂O₃ | 9,0 % | 8,6 | 9,3 |
| K₂O | 10,7 % | 11,2 | 10,2 |
| Na₂O | 10,9 % | 10,7 | 12,0 |
| CaO | 1,0 % | 0,9 | 0,8 |
| BaO | 0,9 % | 0,7 | 0,8 |
| B₂O₃ | 1,0 % | 1,8 | 0,5 |
| Sb₂O₃ | 0,3 % | 0,2 | 0,1 |
| CeO₂ | 0,2 % | 0,3 | 0,1 |
| TiO₂ | 2,1 % | 1,2 | 1,3 |
| Li₂O | 1,0 % | 0,8 | 1,4 |
| F₂ | 1,6 % | 2,7 | 1,2 |

## Patentansprüche

1. Dentalkeramischer Werkstoff zur Herstellung und Reparatur von metallkeramischem und vollkeramischem Zahnersatz mit einer Verarbeitungstemperatur unterhalb 700° C und einem Wärmeausdehnungskoeffizienten α von (13-14)·10⁻⁶·K⁻¹ zwischen 20 und 500° C,
gekennzeichnet durch die Zusammensetzung:
60 bis 65 Gew. % SiO₂, 8,5 bis 11 Gew. % Al₂O₃, 8 bis 12 Gew. % K₂O, 10,5 bis 12 Gew. % Na₂O, 0,7 bis 2 Gew. % CaO, 0,6 bis 2 Gew. % BaO, 0,5 bis 2,5 Gew. % B₂O₃, 0,1 bis 0,6 Gew. % Sb₂O₃, 0 bis 0,5 Gew. % CeO₂, 1,2 bis 3,8 Gew. % TiO₂, 0,8 bis 1,4 Gew. % Li₂O und 1,2 bis 3,8 Gew. % F₂.

2. Dentalkeramischer Werkstoff nach Anspruch 1,
dadurch gekennzeichnet,
daß er 60 bis 63 Gew. % SiO₂, 8,5 bis 9,5 Gew. % Al₂O₃, 10 bis 11,5 Gew. % K₂O, 10,5 bis 11,5 Gew. % Na₂O, 0,7 bis 1,5 Gew. % CaO, 0,6 bis 1,2 Gew. % BaO, 0,7 bis 1,5 Gew. % B₂O₃, 0,2 bis 0,4 Gew. % Sb₂O₃, 0,1 bis 0,4 Gew. % CeO₂, 1,5 bis 3 Gew. % TiO₂, 0,8 bis 1,2 Gew. % Li₂O und 1,2 bis 2,4 Gew. % F₂ enthält.

## Claims

1. Dental ceramic material for the production and repair of cermet and full ceramic dentures, having a processing temperature of below 700°C and a coefficient of thermal expansion α of (13 - 14).10⁻⁶ . K⁻¹ at between 20 and 500°C,
characterised by the composition:
60 to 65% by weight of SiO₂, 8.5 to 11% by weight of Al₂O₃, 8 to 12% by weight of K₂O, 10.5 to 12% by weight of Na₂O, 0.7 to 2% by weight of CaO, 0.6 to 2% by weight of BaO, 0.5 to 2.5% by weight of B₂O₃, 0.1 to 0.6% by weight of Sb₂O₃, 0 to 0.5% by weight of CeO₂, 1.2 to 3.8% by weight of TiO₂, 0.8 to 1.4% by weight of Li₂O and 1.2 to 3.8% by weight of F₂.

2. Dental ceramic material according to claim 1,
characterised in that it contains
60 to 63% by weight of SiO₂, 8.5 to 9.5% by weight of Al₂O₃, 10 to 11.5% by weight of K₂O, 10.5 to 11.5% by weight of Na₂O, 0.7 to 1.5% by weight of CaO, 0.6 to 1.2% by weight of BaO, 0.7 to 1.5% by weight of B₂O₃, 0.2 to 0.4% by weight of Sb₂O₃, 0.1 to 0.4% by weight of CeO₂, 1.5 to 3% by weight of TiO₂, 0.8 to 1.2% by weight of Li₂O and 1.2 to 2.4% by weight of F₂.

## Revendications

1. Matériau céramique dentaire pour la production et la réparation de prothèses dentaires en céramique métallique et en céramique complète ayant une température d'usinage en dessous de 700°C et un coefficient de dilatation thermique α de (13-14)·10⁻⁶·K⁻¹ entre 20 et 500°C, caractérisé par la composition : de 60 à 65 % en poids de SiO₂, 8,5 à 11 % en poids d'A1₂O₃, 8 à 12 % en poids de K₂O, 10,5 à 12 % en poids de Na₂O₇, 0,7 à 2 % en poids de CaO, 0,6 à 2 % en poids de BaO, 0,5 à 2,5 % en poids de B₂O₃, 0,1 à 0,6 % en poids de Sb₂O₃, 0 à 0,5 % en poids de CeO₂, 1,2 à 3,8 % en poids de TiO₂, 0,8 à 1,4 % en poids de Li₂O et 1,2 à 3,8 % en poids de F₂.

2. Matériau en céramique dentaire selon la revendication 1 caractérisé en ce qu'il renferme 60 à 63 % en poids de SiO₂, 8,5 à 9,5 % en poids d'A1₂O₃, 10 à 11,5 % en poids de K₂O, 10,5 à 11,5 % en poids de Na₂O, 0,7 à 1,5 % en poids de CaO, 0,6 à 1,2 % en poids de BaO, 0,7 à 1,5 % en poids de B₂O₃, 0,2 à 0,4 % en poids de Sb₂O₃, 0,1 à 0,4 % en poids de CeO₂, 1,5 à 3 % en poids de TiO₂, 0,8 à 1,2 % en poids de Li₂O et 1,2 à 2,4 % en poids de F₂.
